# EUROPEAN PATENT APPLICATION

(11) **EP 3 342 442 A1**
(43) Date of publication of application: **04.07.2018**
(21) Application number: 16207272.2
(22) Date of filing: 29.12.2016
(51) Int. Cl.: A61M 15/06, A24F 47/00

(54) **AEROSOL GENERATING SYSTEM AND METHOD OF CONTROLLING THE OPERATION OF AN AEROSOL GENERATING SYSTEM**

(71) Applicant: JT International S.A., 1202 Geneva (CH)
(72) Inventor: LAWRENSON, Matthew John, 1030 Bussigny (CH); NOLAN, Julian Charles, 1009 Pully (CH)
(74) Representative: Isarpatent

(57) **Abstract**

An aerosol generating system has a vaporizer, and electrical circuitry configured to selectively activate and deactivate the vaporizer. The aerosol generating system further includes a signal emitter, a signal receiver and an authorization controller. The authorization controller is configured to emit probing signals via the signal emitter, to receive reflected response signals via the signal receiver, to compare a profile of the received reflected response signals with a predetermined response signal profile and to output, based on the comparison, an activation or deactivation signal to the electrical circuitry in order to control an operational state of the vaporizer.

## Description

### Field of the invention

The present invention pertains to an aerosol generating system, and a method for controlling the operation of an aerosol generating system, in particular refillable electronic cigarettes or vaporizers. The aerosol generating system and the corresponding method for controlling the operation thereof may be particularly useful in restricting access to aerosol generating systems based on physiological characteristics of potential users.

### Background of the invention

Electrically operated cigarettes, so-called "e-cigarettes", usually include a heater powered by an electrical power source and a liquid reservoir containing flavoured or unflavoured liquids that can be volatilized using the heater and transferred to a user of the e-cigarette in an airflow through a mouthpiece of the e-cigarette. Such an electrically operated cigarette is for example known from the document US 2013/0160764 A1.

E-cigarettes may employ biologically and/or physiologically active substances comprised in the liquids which need to be handled with specific care and caution. There is a need to prevent operation of the e-cigarette by a limited group of persons and it would be desirable to develop e-cigarettes with inherent means to restrict access thereto to underage users for example underage user.

Document US 2015/0181945 A1 discloses electronic cigarettes that may be controlled on buccal biometric data obtained via image processing.

Documents US 2014/0246035 A1 and US 2016/0021930 A1 disclose personal vaporizer devices which may be remotely activated or deactivated using biometric authentication methods.

Document US 2015/0136158 A1 discloses methods for managing and preventing access of minors or unapproved consumers to administration of controlled substances using portable electronic vaporization devices.

### Summary of the invention

It is therefore one of the objects of the present invention to provide solutions for aerosol generating systems that are able to prevent unsolicited operation by an underage user but at the same time allow for convenient usage of the aerosol generating system by adults.

This problem is solved by an aerosol generating system having the technical features of claim 1 and a method for controlling the operation of an aerosol generating system having the technical features of claim 12.

According to a first aspect of the present invention, an aerosol generating device comprises a vaporizer, and electrical circuitry configured to selectively activate and deactivate the vaporizer. The aerosol generating device further comprises a signal emitter, a signal receiver and an authorization controller. The authorization controller is configured to emit probing signals via the signal emitter, to receive reflected response signals via the signal receiver, to compare a profile of the received reflected response signals with a predetermined response signal profile and to control, based on the comparison, the electrical circuitry in order to control an operational state of the vaporizer. The electrical circuitry may for example be controlled by outputting an activation or deactivation signal by the authorization controller to the electrical circuitry.

According to a second aspect of the present invention, a method for controlling the operation of an aerosol generating system comprises emitting an electroacoustic reference signal via an electroacoustic transmitter of the aerosol generating device into an oral cavity of a user of the aerosol generating system and receiving reflected response signals of the emitted electroacoustic reference signal via an electroacoustic receiver of the aerosol generating system from the oral cavity of the user. A profile of the received reflected response signals is compared with a predetermined response signal profile stored in the aerosol generating system. Based on the result of the comparison, electrical circuitry of the aerosol generating system is controlled, for example by outputting an activation or deactivation signal to the electrical circuitry, in order to control an operational state of a vaporization mechanism of the aerosol generating system, which vaporizer is configured to volatilize compounds of a liquid or gas from a storage component of the vaporizer into an airflow streaming through the aerosol generating system. In a further aspect of the present invention, electrical circuitry for an electrically operated aerosol generating system is configured to implement the method of the second aspect of the present invention.

According to a third aspect of the invention, a method includes machine executable instructions that enable a machine to perform the following operations: Emitting an electroacoustic reference signal via an electroacoustic transmitter of the aerosol generating device into an oral cavity of a user of the aerosol generating system and receiving reflected response signals of the emitted electroacoustic reference signal via an electroacoustic receiver of the aerosol generating system from the oral cavity of the user. A profile of the received reflected response signals is compared with a predetermined response signal profile stored in the aerosol generating system. Based on the result of the comparison, electrical circuitry of the aerosol generating system is controlled, for example by outputting an activation or deactivation signal to the electrical circuitry, in order to control an operational state of a vaporization mechanism of the aerosol generating system, which vaporizer is configured to volatilize compounds of a liquid or gas from a storage component of the vaporizer into an airflow streaming through the aerosol generating system. The method according to the third aspect of the invention may be stored on a tangible, i.e. non-transitory computer readable medium which includes instructions which enable a machine to perform the operations of the method according to the third aspect of the invention.

According to a fourth aspect of the invention, a computer program may include machine-readable code that is designed to perform the operations of the method according to the third aspect of the invention when compiled and run on a computer. The computer program according to the fourth aspect of the invention may be stored on a tangible, i.e. non-transitory computer readable medium which includes instructions which enable a computer to run the computer program according to the fourth aspect of the invention.

As used herein, the term "computer readable medium/media" may include conventional non-transient memory, for example one or more of: random access memory (RAM); a CD-ROM; a hard drive; a solid state drive; a flash drive; a memory card; a DVD-ROM; a floppy disk; an optical drive. The memory may have various arrangements corresponding to those discussed for the circuitry/processor.

### Embodiments of the invention

One main idea of the present invention is to provide an aerosol generating system the activation of the aerosol generation mechanism of which is controlled by an authorisation component that injects signals, such as acoustic, particularly ultrasonic, signals into the oral cavity of a current user of the aerosol generating device and measures a corresponding response. By analyzing characteristics of the response signal certain physiological characteristics of (parts of) the oral cavity may be at least qualitatively determined. Depending on the comparison of the determined physiological characteristics with predetermined thresholds or signal profiles, the authorisation component may then selectively activate or deactivate the aerosol generation mechanism. The predetermined thresholds or signal profiles are based on likelihood values indicating a likelihood that the current user of the aerosol generating system is a certain user or at least a user belonging to certain group of user having similarly physiological characteristics.

For example, a distinction may be made between groups of users having the physiological characteristics of underage user or of adults. The authorisation component is therefore able to activate the aerosol generation mechanism only if the likelihood that the current user of the aerosol generating system is an adult is sufficiently high.

In other cases, certain individuals may be identified on the basis of their physiological characteristics and the characteristic response signals being generated upon injection of acoustic signals into their oral cavity. The authorisation component may then be able to activate the aerosol generation mechanism only if the likelihood that the current user of the aerosol generating system is the intended user, for example the registered user of the particular aerosol generating system.

With an aerosol generating system according to the invention, tampering of the aerosol generating system by underage user may be advantageously prevented or at least impeded, thus safeguarding the underage user's health and wellbeing. Moreover, in some cases it may be possible differentiate between various users and thus provide an authentication mechanism based on the biometric properties of the intended user.

The operational mode of the aerosol generating system, and particularly of the vaporizer, may be chosen dependent on physiological features of human users, thereby achieving the purpose of implementing a way of controlling the operation of the aerosol generating system that is difficult to circumvent, be it with or without malicious intent.

The disclosed aerosol generating systems and the methods for controlling the operation thereof are advantageously able to provide for e-cigarette products that comply with the Tobacco Products Directive 2014/40/EU of the European Parliament and the European Council. The embodiments of aerosol generating systems as disclosed herein are child- and tamperproof, specifically due the protection mechanisms against uncontrolled or unsolicited access.

The disclosed aerosol generating systems and the corresponding methods for controlling the operation thereof allow for a convenient and automatic manner of restricting access to the aerosol generating system. The rules of such access restriction and the authorization assessment carried out by the aerosol generating system may even be made transparent to the user, further enhancing the convenience of use.

The dependent claims provide additional technical features of advantageous embodiments and further improvements of the invention.

According to an embodiment of the aerosol generating system, the aerosol generating system may further comprise a power source configured to supply the vaporizer with electrical energy. In a specific embodiment, the vaporizer may further comprise a heating element coupled to the power source.

According to another embodiment of the aerosol generating system, the aerosol generating system may comprise a device body that may comprise a battery housing section and a vaporising housing section connected to the battery housing section. In a specific embodiment, the battery housing section may house the power source. In a further specific embodiment, the vaporising housing section may house the vaporizer.

According to another embodiment of the aerosol generating system, the system may further comprise an airflow activation sensor coupled to the authorization controller. In some embodiments, the airflow activation sensor may be configured to measure an airflow parameter of an airflow streaming through the aerosol generating system and to activate the authorization controller when the measured airflow parameter exceeds a predetermined threshold value.

According to yet another embodiment of the aerosol generating system, the system may further comprise an authorization trigger module coupled to the authorization controller, the authorization trigger module being configured to detect a user-initiated activation condition for the aerosol generating system and to activate the authorization controller upon detection of the user-initiated activation condition.

In some specific embodiments, such an authorization trigger module may include a mechanical activation element, for example an element arranged in or on a device body of the aerosol generating system. In some embodiments, the user-initiated activation condition may include an actuation of such a mechanical activation element by a user of the aerosol generating.

According to further embodiments of the aerosol generating system, the authorization trigger module may include an acceleration sensor or a position sensor In some embodiments, the acceleration sensor or position sensor may be configured to detect a movement or a position of the aerosol generating system, and the user-initiated activation condition may include a detected movement or position of the aerosol generating system corresponding to one of a number of predefined movement profiles or position profiles of the aerosol generating system.

According to other embodiments of the aerosol generating system, the authorization trigger module may include a proximity sensor arranged in the system. In some embodiments, the proximity sensor may be configured to detect a contact of the aerosol generating system with a body part of a user, and the user-initiated activation condition may include a detected contact of the aerosol generating system with a body part of a user.

According to another embodiment of the aerosol generating system, the signal emitter may comprise an electroacoustic transmitter, an optical emitter or a radar emitter. In some corresponding embodiments, the signal receiver may comprise an electroacoustic receiver, an optical receiver or a radar receiver.

According to another embodiment of the aerosol generating system, the authorization controller may include an authorization processor and a data storage coupled to the authorization processor. In some embodiments, the data storage may be configured to store a plurality of predetermined response signal profiles. In some specific embodiments, the authorization processor may be configured to measure one or more of a frequency of the reflected response signals, a distribution of frequencies of the reflected response signals, and an amplitude of the reflected response signals at a predefined frequency or a predefined set of frequencies.

According to an embodiment of the method, the method may further involve detecting a user-initiated activation condition for the aerosol generating system. In some embodiments, emitting of the electroacoustic reference signal may then be triggered upon detection of the user-initiated activation condition. In some specific embodiments of this method, the activation or deactivation signal outputted to the vaporization controller may be maintained until a change in the user-initiated activation condition is detected.

The invention will be explained in greater detail with reference to exemplary embodiments depicted in the drawings as appended.

### Brief description of the drawings

The accompanying drawings are included to provide a further understanding of the present invention and are incorporated in and constitute a part of this specification.

The drawings illustrate the embodiments of the present invention and together with the description serve to explain the principles of the invention. Other embodiments of the present invention and many of the intended advantages of the present invention will be readily appreciated as they become better understood by reference to the following detailed description. The elements of the drawings are not necessarily to scale relative to each other. Like reference numerals designate corresponding similar parts.
Fig. 1 schematically illustrates a functional depiction of an aerosol generating system according to an embodiment of the invention.
Fig. 2 schematically illustrates a possible implementation of an authorisation component of the aerosol generating system of Fig. 1 according to further embodiments of the invention.
Fig. 3 schematically illustrates an event sequence diagram depicting a possible procedure for verifying physiological characteristics of a user of an aerosol generating system according to yet another embodiment of the invention.
Fig. 4 schematically illustrates a method for controlling an operation of an aerosol generating system according to yet another embodiment of the invention.

### Detailed description of the embodiments

Although specific embodiments have been illustrated and described herein, it will be appreciated by those of ordinary skill in the art that a variety of alternate and/or equivalent implementations may be substituted for the specific embodiments shown and described without departing from the scope of the present invention.

Generally, this application is intended to cover any adaptations or variations of the specific embodiments discussed herein. In the present invention, reference is made to aerosol generators. Such aerosol generators or aerosol generating devices are generally intended to comprise any apparatus capable of converting electric energy and/or combustion energy into heat and subsequently heating and thereby volatilizing particles in a vaporisable material, for example, a liquid or gaseous composition contained within a part of the aerosol generating device. Aerosol generating devices within the meaning of the present invention may transport the volatilized particles in an airflow through the aerosol generating device to a user of the device, the user of the device being able to activate or deactivate the generation of aerosol and to control the duration, velocity and volume of the airflow by means of puffing or inhaling action.

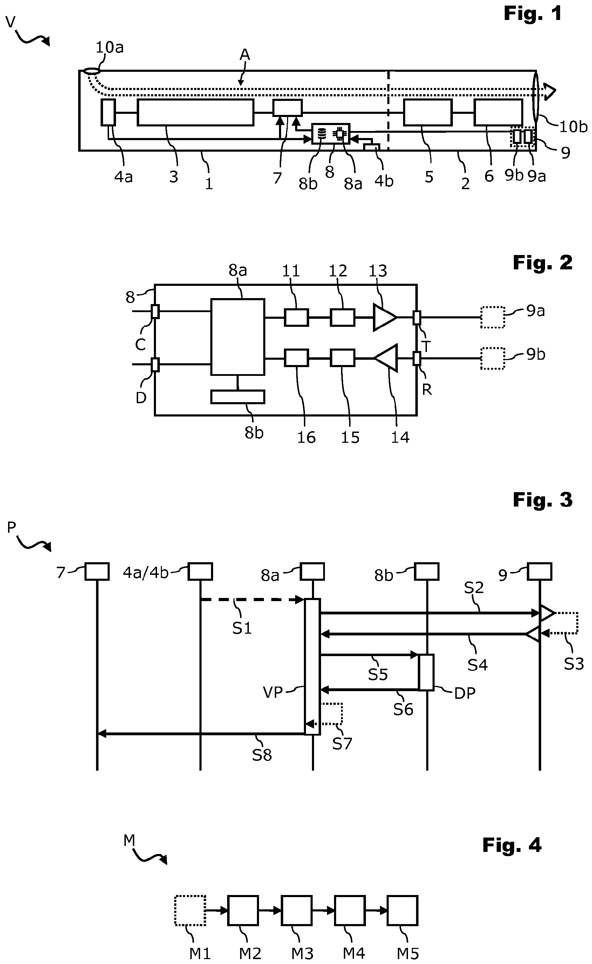

Fig. 1 schematically illustrates a functional depiction of an aerosol generating system or device V. The aerosol generating system V may comprise a single device as exemplarily depicted in the drawings, or may alternatively comprise a system of distributed components. As illustrated in Fig. 1, the aerosol generating device V may generally comprise a housing. The housing may be formed as integral hollow body. It may also be possible for the housing to have a battery housing section 1 and a vaporising housing section 2, such as the ones exemplarily depicted in Fig. 1, which together form a generally hollow body of the aerosol generating device V and are fluidly connected to each other.

The battery housing section 1 may house a power source 3, such as an electrical power source, for example a battery or an accumulator. The battery housing section 1 may also comprise an activation sensor system 4a that is configured to selectively activate electrical circuitry acting as a vaporization controller 7 between the power source 3 and components of the vaporising housing section 2. The battery housing section 1 and the vaporising housing section 2 may together form a substantially cylindrical device body of the aerosol generating device V. However, it should be appreciated that any other outer shape, for example prismatic or ellipsoid shapes may be suitable for the battery housing section 1 and the vaporising housing section 2 as well.

The vaporising housing section 2 comprises a vaporizer, which may for example comprise an atomizer and a mouthpiece as separate components. The atomizer may for example comprise a heating element 5 electrically connected to the power source 3, a liquid storage component 6, and a means for providing a liquid interface between the heating element 5 and liquid storage component 6 such as a wick. The vaporising housing section 2 may also comprise a cartomizer, i.e. an integral combination of an atomizer and a liquid storage component embedded in a hollow housing, the end of the hollow housing or housing section forming a mouthpiece having an airflow outlet 10b.

As an exemplary configuration, Fig. 1 depicts a vaporising housing section 2 comprising a heating element 5, for exampe a nichrome wire coil, which is coupled to the power source 3 via the electrical circuitry of the vaporization controller 7. The heating element 5 is supplied with energy for its operation by the power source 3 when activated by the vaporization controller 7. Fig. 1 further exemplarily depicts a liquid storage component 6 fluidly coupled to the heating element 5 in operative connection so that the heating element 5 may supply heating energy to a liquid or gas contained with the liquid storage component 6, thereby causing certain compounds contained in the liquid to volatilize into an airflow A streaming through the vaporising housing section 2 from one or more airflow inlets (schematically depicted as 10a in Fig. 1) to the airflow outlet 10b. The vaporising housing section 2 may have a mouthpiece with an airflow outlet, generally depicted as reference numeral 10b, through which aerosol generated from the liquid storage component 6 may be guided towards a user of the aerosol generating device V performing an inhaling action at the mouthpiece.

The liquid storage component 2 may comprise a liquid reservoir in which a liquid composition with compounds to be volatilized may be contained or a sponge wetted with such a liquid. The liquid storage component 6 may be in connection by means of a liquid interface with the heating element 5 which may convey heating energy to the liquid, thereby evaporating or volatilizing certain compounds of the liquid. The liquid interface between the heating element 5 and the liquid storage component 6 may result from the heating element 5 forming an electrode plunged into the liquid reservoir or, in a more common alternative, from a liquid transfer arrangement such as a wick extending between the liquid storage component 6 and the heating element 5.

The airflow A to be inhaled by the user of the aerosol generating device V may flow through an airflow channel extending partially through the liquid storage component 6 or around it. Thereby, upon an air drawing action of the user at the mouthpiece an aerosol is generated in the aerosol generating device V due to the low pressure created by puffing at the mouthpiece and the aerosol can be inhaled by the user from the airflow outlet 10b at the mouthpiece.

Air being drawn in by a user through an inhaling action at the mouthpiece 10 will cause an airflow A streaming in the proximity of the activation sensor system 4a. The activation sensor system 4a is configured to detect a variation of at least one parameter of this airflow such as pressure, velocity, airflow rate near an active sensor surface. For example, the activation sensor system 4a may comprise a mechanical-to-electrical transducer that is able to produce an electrical signal depending on detected variations in air pressure in its vicinity. The activation sensor system 4a may for example comprise a microphone, such as a dynamic microphone, condenser microphone, capacitance microphone or piezoelectric microphone. It may be possible to employ micro-electromechanical systems (MEMS) activation sensors 4a. Alternatively, other types of activation sensor systems 4a may also be used such as pressure differential sensors, mass flow sensors, velocity sensors, flow rate sensors, temperature sensors or the like. It may also be possible to employ multiple types of sensors in the activation sensor system 4a and to combine their measurement values to a consolidated detection result which may then be compared to a combined activation threshold.

The activation sensor system 4a may have an activation threshold (or combined activation threshold for multiple sensors) for selectively causing the electrical circuitry of the vaporization controller 7 to activate the supply of the heating element 5 with power of the power source 3. When the vacuum pressure, i.e. the drop in pressure in the vicinity of the active sensor surface of the activation sensor 4a surpasses the activation threshold, the activation sensor 4a will send an activation signal to the vaporization controller 7, thereby causing an electrical connection between the power source 3 and the heating element 5 to be established and allowing the heating element 5 to be powered by the power source 3.

The aerosol generating device V may further comprise electronic circuitry controlling the operation of the aerosol generating device V and/or various buttons or light emitting elements on its outer surface, all of which are not shown in Fig. 1 for purposes of improved clarity of the drawings.

The battery housing section 1 and the vaporising housing section 2 may be separable, for example by means of a connector that is arranged in the vaporising housing section 2 in order to mechanically and releasably connect the vaporising housing section 2 to the battery housing section 1. The connector and the connector receptacle of the sections 1 and 2 may be constructed in a complementary and corresponding manner, for example by a moulding procedure, to realize complementary snap-fit parts or interlocking clearances. For example, the connector may include protrusions that interlock with recesses in the connector receptacle when edging the connector into the connector receptacle. Moreover, the connector and the connector receptacle may be designed with electrical connections being formed between the power source 3 and the heating element 5 upon connecting the battery housing section 1 and the vaporising housing section 2.

It may further be possible to provide sealing means, such as a rubber O-ring or similar, between the connector and the connector receptacles in order to fluidly seal the interface between the battery housing section 1 and the vaporising housing section 2 against the environment.

With respect to Fig. 1, the function of the aerosol generating system or device V will now be described in further detail. The aerosol generating system or device V comprises an authorization controller 8 that is coupled to the vaporization controller 7 on one hand and may receive input signals from the activation sensor system 4a, an authorization trigger module 4b and/or an electroacoustic transducer module 9 on the other hand. The authorization controller 8 may be implemented as a separate component to the electrical circuitry forming the vaporization controller. Of course, it may equally be possible to at least partially or wholly implement circuitry forming the authorization controller 8 in the electrical circuitry forming the vaporization controller 7, i.e. the authorization controller 8 and the vaporization controller 7 may be formed with at least partially common electrical or electronic components that are designed to fulfil both the functions of the authorization controller 8 and the vaporization controller 7.

Upon activation, the authorization controller 8 is generally configured to perform an authentication or authorization assessment procedure, i.e. the authorization controller 8 is able to determine whether a particular current user of the aerosol generating device V is (or should be) authorized to operate the aerosol generating device V, and particularly the vaporization mechanism thereof.

The activation of the authorization controller 8 may in particular depend on input signals generated by various triggering components such as the activation sensor system 4a and/or the authorization trigger module 4b.

In some configurations, the activation sensor system 4a doubles as triggering component for the activation of the vaporization controller 7 per se and as triggering component for the activation of the authorization controller 8. For example, when an airflow A through the aerosol generating device V is detected by the activation sensor system 4a, the authorization controller 8 is activated by a triggering signal sent from the activation sensor system 4a to the authorization controller 8. For example, the activation sensor system 4a may be configured to measure an airflow parameter of an airflow A streaming through the aerosol generating device V. Once such measured airflow parameter exceeds a predetermined threshold value, the activation sensor system 4a may activate the authorization controller in order to cause the authorization controller 8 to perform an authorization assessment procedure. After performing its authorization assessment procedure, the authorization controller 8 sends an additional activation signal to the vaporization controller 7. The vaporization controller 7 will then only activate the supply of the heating element 5 with power of the power source 3 when both the activation sensor system 4a and the authorization controller 8 send their respective activation signals. This causes an authorization assessment procedure to be performed every time when a user tries to take a draw on the mouthpiece of the aerosol generating device V, i.e. when a user tries to initiate an aerosol generating procedure within the aerosol generating device V and to subsequently inhale the aerosol created by the aerosol generating device V.

In some other configurations, the authorization controller 8 may (additionally or alternatively to the triggering signals from the activation sensor system 4a) receive input signals from the authorization trigger module 4b coupled to the authorization controller 8. The authorization trigger module 4b is in general configured to detect one or more user-initiated activation conditions for the aerosol generating device V. Such user-initiated activation conditions may comprise other conditions than the detection on the presence of an airflow A through the aerosol generating device V. When such user-initiated activation condition is detected, the authorization trigger module 4b sends an activation signal to the authorization controller 8 in order to trigger the operation of the authorization controller 8.

The authorization trigger module 4b may include various different components: For example, a mechanical activation element, such as a pushbutton or a touch-sensitive interface element, may be arranged in or on the device body. A user-initiated activation condition may then include an actuation of the mechanical activation element by a user, for example pushing the button or touching the interface element.

The authorization trigger module 4b may also be formed as acceleration sensor or position sensor. Those sensors may be configured to detect movements or positional information regarding the aerosol generating device V. If such a movement or positional information is detected and corresponds to a certain predetermined movement or position, the authorization trigger module 4b may trigger the operation of the authorization controller 8. In particular, the movements and/or positions of the aerosol generating device V as detected by the authorization trigger module 4b may be compared to specific movement profiles or position profiles which indicate that an authorization assessment procedure might be warranted.

The authorization trigger module 4b may also be formed as a proximity sensor arranged in the device body. Such proximity sensors may detect contacts of the aerosol generating device V with one or more body parts of a user, for example taking the device into the hands or passing the device on to another user. The user-initiated activation condition may in this case include a detected contact with a body part of a user corresponding to one of a number of specified contacts which indicate that an authorization assessment procedure might be warranted, or simply the presence of a contact with a body part of a user at all.

The operation of the authorization controller 8 generally involves emitting a probing signal, such as an electroacoustic reference signal via a signal emitter 9a. The signal emitter may be an electroacoustic transmitter 9a. The electroacoustic transmitter 9a may be arranged in the device body and may in some cases be integrated into the electroacoustic transducer module 9. The electroacoustic reference signal may for example be an ultrasonic reference signal. Such an ultrasonic reference signal may be emitted by the electroacoustic transmitter 9a into an oral cavity of a user of the aerosol generating device V. To that end, the electroacoustic transmitter 9a may be located or arranged near the mouthpiece of the aerosol generating device V. The electroacoustic transmitter 9a may for example be embodied as a loudspeaker that is capable of emitting acoustic waves in the desired frequency range, such as for example ultrasound.

Electroacoustic reference signals emitted into an oral cavity of a user may for example cause resonance of the jaw bone and/or the teeth of the user. Resonance may occur where the electroacoustic reference signal as external stimulus drives a system to oscillate with greater amplitude at a specific frequency. The shape, density and other acoustic properties of the jaw bone and/or the teeth of the user exhibit specific resonance frequencies that may be excited by corresponding frequencies of the electroacoustic reference signal.

Alternatively or additionally, such electroacoustic reference signals may cause a standing wave to be established in the user's respiratory tract, including in some cases the nasal cavity, the larynx, the trachea and the bronchi. Standing waves may occur when the reflection of a wave interferes with the original wave such that the nodes of the resultant wave are stationary, i.e. when the original wave and the reflected wave are at least partially in phase.

The electroacoustic reference signals may vary in frequency over a frequency range, i.e. the electroacoustic reference signals may have a certain pre-defined spectral distribution. It may also be possible to tune the frequency over time during emission of the electroacoustic reference signals. Moreover, it may be possible for the electroacoustic reference signals to contain a series of differently toned signals in a signal sequence. When the electroacoustic reference signals enter the oral cavity, the jawbone and/or teeth within the jawbone may resonate. Additionally, a standing wave may be set up within the user's respiratory tract.

A signal receiver 9b, such as an electroacoustic receiver 9b, of the aerosol generating device V that may be arranged in the device body may be used to receive any response signals. For example, it may be possible to receive reflexions of the emitted electroacoustic reference signals as response signals. The received reflected response signals may be relayed from the electroacoustic receiver 9b to the authorization controller 8 for further proecssing. The electroacoustic receiver 9b may for example be embodied as a microphone that is capable of receiving acoustic waves in the desired frequency range, such as for example ultrasound. The electroacoustic receiver 9b and transmitter 9a may both be arranged within a common electroacoustic transducer module 9. It may of course also be possible to implement the electroacoustic receiver 9b and the electroacoustic transmitter 9a as separate components within the aerosol generating device V.

By evaluating the parameters of the received reflected response signals it may be possible to draw conclusions to size, shape and/or other geometrical properties of (parts of) the oral cavity of the user. Thus, it may be possible to deduce whether the user is a child or an adult, as underage user will usually have smaller jawbones, teeth and respiratory tracts. It is not necessarily intended to provide an accurate assessment of the user's age, but the significantly different anatomies of underage user as compared to an adult with respect to the mentioned body parts may allow for a sufficiently accurate distinction between underage user and adults.

For standing waves to be created in the respiratory tract, the aerosol generating device V should preferably be placed such that acoustic waves emitted by the electroacoustic transmitter 9a can enter the mouth. Additionally, if the vocal chords are in an open position, the volume for the standing wave may be advantageously enlarged, as the volume of the mouth alone is small and the shape of the oral cavity alone may vary significantly as the tongue is moved within it. One way to ensure that the vocal chords are open may be to have the user inhale during the assessment procedure performed by the authorization controller 8. Usually, the method of use of an aerosol generating device V is to draw air through it and then into the user's lungs. Hence, by carrying out the assessment at the start of this sequence the interaction with the aerosol generating device V may remain familiar to the user.

The assessment of resonant frequencies of the jawbones and/or teeth do not necessarily require the vocal chords to be open, however, the aerosol generating device V may advantageously be placed such that the acoustic waves emitted by the electroacoustic transmitter 9a can enter the mouth. During intended use, the aerosol generating device V is usually in contact with the user's lips or teeth so that the coupling between the reflected response signals and the electroacoustic receiver 9b is sufficiently high.

The profile of the reflected response signals is compared by the authorization controller 8 with a predetermined response signal profile. This predetermined response signal profile may account for typical differences in resonance frequencies, resonance amplitudes and/or other spectral parameters of acoustic waves between underage user and adults. Spectral parameters to be taken into account for purposes of profile comparisons may be a frequency of the reflected response signals, a distribution of frequencies of the reflected response signals, and amplitudes of the reflected response signals at predefined frequencies or a predefined set of frequencies. Based on the result of the comparison, an activation or deactivation signal may be outputted by the authorization controller 8 to the vaporization controller 7. That way, the vaporization controller 7 may be controlled to set different operational states of the vaporization mechanism of the aerosol generating device V.

Aerosol generating devices such as electronic cigarettes usually operate energy efficiently, so that the device generally powers down when not in use. This includes the operation of the authorization controller 8 as well. Therefore, a 'pre-use' condition may be implemented, i.e. a condition that needs to be met in order to power the authorization controller 8. For example, a pre-use condition may involve the press of a button on the device, or the user sucking air through the device. Once such a condition is detected, the authorization controller 8 may be automatically powered up.

The decision of the authorization controller 8 whether to instruct the vaporization controller 7 to activate or deactivate the vaporization mechanism may be upheld for an amount of time that may be set depending on desired functionality. For example, the activation or deactivation signal output to the vaporization controller 7 by the authorization controller 8 may persist depending on a fixed and predetermined amount of time (for example 1 minute, 2 minutes or 10 minutes or any other given timespan), on the time period between two intended inhalations by the user, on the detection by an appropriate sensor (position sensor, touch sensor or similar) that the aerosol generating device V had been put down or had been given to another user (for example by detecting that the number of fingers holding the device has changed or the grip has changed), on an alotted number of draws between two consecutive authorization assessment procedures or on other boundary conditions.

Fig. 2 exemplarily depicts a possible implementation of an authorization controller, such as the authorization controller 8 as shown in Fig. 1. The authorization controller 8 may generally include a transmitter interface T coupled to the electroacoustic transmitter 9a, a receiver interface R coupled to the electroacoustic receiver 9b, and an authorization output interface C via which the authorization controller 8 may output activation or deactivation signals to the vaporization controller 7 in order to control the operation of the vaporizer.

Optionally, the authorization controller 8 may also include an authorization trigger interface D via which an activation sensor system 4a or an authorization trigger module 4b (or both) may be coupled to the authorization controller 8. The authorization controller 8 may in those cases only be triggered to operate, i.e. to perform an authorization assessment procedure as for example explained in conjunction with Fig. 3 below when it is activated by a corresponding trigger signal from the activation sensor system 4a and/or the authorization trigger module 4b.

The authorization controller 8 generally includes an authorization processor 8a and a data storage 8b coupled to the authorization processor 8a. The authorization processor 8a may for example be a microprocessor, a DSP, an ASIC, an FPGA, a PLD or any other suitable programmable logic unit. The authorization processor 8a may in particular be configured to run software, firmware or other executable code enabling it to perform authorization assessment procedures as described hereinbelow. The data storage 8b may in particular be configured to store a plurality of predetermined response signal profiles. Further, the data storage 8b may contain software, firmware or configuration data necessary for the operation of the authorization processor 8a. The data storage 8b may for example be a random access memory, a read-only memory, a flash drive, a solid-state drive or any other suitable memory component. It may also be possible for the data storage 8b and the authorization processor 8a to be embodied in a single integrated component, such as a microcontroller.

The authorization processor 8a may be coupled to the transmitter interface T via a digital-to-analog converter 11 and a low-pass filter 12. The authorization processor 8a may output a digital reference signal for emission by the electroacoustic transmitter 9a which is first converted to an analog signal by the analog converter 11 and then filtered by the low-pass filter 12. The filtered analog reference signal may then be amplified using an amplifier 13 and passed on to the electroacoustic transmitter 9a via the transmitter interface T.

Correspondingly, response signals received from the electroacoustic receiver 9b via the receiver interface R may be amplified using the amplifier 14. The amplified response signals may be passed on to an envelope detector circuit 15 that detects envelopes of the amplified response signals and passes the detected envelopes on to an analog-to-digital converter 16 which in turn outputs the digital received response signals to the authorization processor 8a for further processing. The envelope detector 15 may for example include a diode detector circuit.

Fig. 3 schematically illustrates an event sequence diagram P in which the procedure for verifying physiological characteristic of a user of an aerosol generating device, such as the aerosol generating device V as illustrated in and explained in conjunction with Fig. 1 to 2, is explained. The procedure may involve several events, denoted S1 to S8 in Fig. 3, which may comprise certain components of the aerosol generating device exchanging messages, requests or responses among each other. It is to be understood that the procedure as depicted in Fig. 3 is only of exemplary nature and that the sequence of events or messages may be changed as appropriate or that some events may be left out or effectively merged with other events.

Upon triggering the authorization controller 8 with a first optional triggering signal S1, the authorization controller 8 may initiate an authorization assessment algorithm VP The authorization controller 8 emits a reference signal S2 to the electroacoustic transducer module 9 which in turn projects an analog electroacoustic reference signal S3 into the user's oral cavity. The analog electroacoustic reference signal S3 may be a periodic acoustic wave, for example with an initial frequency f0. The amplitude of the analog electroacoustic reference signal S3 may be selected such that it is sufficient to create a response signal S4 that may be picked up by the electroacoustic receiver 9b. The received response signal S4 should provide a sufficient dynamic range for the authorization controller 8 to adaequately determine its properties.

The frequency of the analog electroacoustic reference signal S3 may be tuned from the lower frequency limit f0 to an upper frequency limit fmax. The lower and upper frequency limits, f0 and fmax, may be defined such that the resonant frequencies of all possible users (authorized and non-authorized) may be identified. The frequency range used, i.e. the frequency range between f0 and fmax, may be based on the fundamental frequency or any harmonic of the fundamental frequency. The time interval for each frequency increment may be selected to render the injection of the acoustic signal as unobtrusive as possible to the user, but should be such that a measurement can be made at each frequency to determine if the fundamental frequency or one of its harmonics has been reached. The sampling rate and output filter of the analog-to-digital converter 16 employed in the response path (cf. Fig. 2) may be selected such that an accurate sine wave may be generated with known amplitude properties between the frequencies f0 and fmax.

The received response signal S4 is amplified, processed by an envelope detector, and then sampled by the analog-to-digital converter 16. The authorization controller 8 controlling the current injection frequency of the analog electroacoustic reference signal S3 compares the detected amplitude (received from the electroacoustic receiver 9b) with the frequency of the reference signal S2 and records the frequency of the sine wave at which the peak magnitude is detected. This frequency is determined to be the resonant frequency (or any harmonic) of the structures in the oral cavity of the user (such as the jawbones, respiratory tract and/or teeth).

A comparison S5 is then made with predefined frequency profiles DP in the data storage 8b by the authorization processor 8a. Those predefined frequency profiles DP may be selected and pre-configured to distinguish the user from being a child or an adult. Depending on the result S6 of the comparison, an authorization flag S7 may be internally set to TRUE or FALSE within the authorization processor 8a. If the authorization flag S7 is set to TRUE, the authorization processor 8a emits an activation signal S8 to the vaporization controller 7 indicating that the user is an authorized user and the vaporization controller 7 may activate the vaporization mechanism of the aerosol generating device V. On the other hand, if the authorization flag S7 is set to FALSE, the authorization processor 8a emits a deactivation signal S8 to the vaporization controller 7 indicating that the user is not an authorized user and the vaporization controller 7 may not activate the vaporization mechanism of the aerosol generating device V. The vaporization controller 7 then enables or disables the functionality to allow the aerosol generating device V to be used (or not be used, respectively) for its primary purpose. This authorization persists until a stipulated condition occurs.

Fig. 4 schematically illustrates a method M for controlling the operation of an aerosol generating system, such as the aerosol generating device V as illustrated in and explained in conjunction with Fig. 1 to 2. The method M may advantageously take into account the event sequence in the communication between components of the aerosol generating device V as schematically illustrated in conjunction with Fig. 3.

The method M may comprise as a first step M1 detecting a user-initiated activation condition for the aerosol generating device V. This first step M1 is optional, and serves to trigger a second step M2 of the method M. The second step M2 involves emitting an electroacoustic reference signal via an electroacoustic transmitter 9a of the aerosol generating device V into an oral cavity of a user of the aerosol generating device V.

In a third step M3, reflected response signals of the emitted electroacoustic reference signal are received via an electroacoustic receiver 9b of the aerosol generating device V from the oral cavity of the user. A profile of the received reflected response signals is compared in a step M4 with a predetermined response signal profile stored in the aerosol generating device V, for example in a data storage 8b of an authorization controller 8. Based on the result of the comparison, an activation or deactivation signal is outputted to a vaporization controller 7 of the aerosol generating device V in step M5. The activation or deactivation signal serves to control an operational state of a vaporizer of the aerosol generating device V, for example the vaporizer as exemplarily depicted in Fig. 1. In particular, the activation or deactivation signal outputted to the vaporization controller 7 may be maintained until a change in the user-initiated activation condition is detected.

With the methods for controlling the operation of an aerosol generating system and with the aerosol generating devices as illustrated herein, it is possible to implement an easy, fairly inexpensive, automatically operable and virtually invisible childproofing solution for e-cigarettes which should be able to pass regulatory tests under the Tobacco Products Directive.

In the foregoing detailed description, various features are grouped together in one or more examples or examples with the purpose of streamlining the disclosure. It is to be understood that the above description is intended to be illustrative, and not restrictive. It is intended to cover all alternatives, modifications and equivalents. Many other examples will be apparent to one skilled in the art upon reviewing the above specification.

The embodiments were chosen and described in order to best explain the principles of the invention and its practical applications, to thereby enable others skilled in the art to best utilize the invention and various embodiments with various modifications as are suited to the particular use contemplated. In the appended claims and throughout the specification, the terms "including" and "in which" are used as the plain-English equivalents of the respective terms "comprising" and "wherein," respectively. Furthermore, the terms "a" and "an" used herein are intended to be understood as meaning one or more unless explicitly stated otherwise. Moreover, the terms "first", "second", "third", etc. are used merely as labels, and are not intended to impose numerical requirements on or to establish a certain ranking of importance of their objects. Terminology used hereinabove to specify geometric or spatial orientation such as "left", "right", "top", "bottom", "side", "front", "back" and the like is not intended to impose any specific limitation as to the orientation of the objects denoted but rather serve to more easily identify the respective features in the drawings.

### List of Reference Signs

- 1: Battery housing section
- 2: Vaporising housing section
- 3: Power source
- 4a: Airflow activation sensor
- 4b: Authorization trigger module
- 5: Heating element
- 6: Liquid storage component
- 7: Vaporization controller
- 8: Authorization controller
- 8a: Authorization processor
- 8b: Data storage
- 9: Electroacoustic transducer module
- 9a: Electroacoustic transmitter
- 9b: Electroacoustic receiver
- 10a: Airflow inlet
- 10b: Airflow outlet
- A: Airflow
- C: Authorization output interface
- D: Authorization trigger interface
- M: Method
- M1: Method step
- M2: Method step
- M3: Method step
- M4: Method step
- M5: Method step
- P: Event sequence diagram
- R: Receiver interface
- S1: Event
- S2: Event
- S3: Event
- S4: Event
- S5: Event
- S6: Event
- S7: Event
- S8: Event
- T: Transmitter interface
- V: Aerosol generating system

## Claims

1. An aerosol generating system (V), comprising:
a vaporizer (5; 6);
electrical circuitry (7) configured to activate and deactivate the vaporizer (5, 6);
a signal emitter (9a);
a signal receiver (9b); and
an authorization controller (8) configured to emit a probing signal via the signal emitter (9a), to receive reflected response signal via the signal receiver (9b), to compare a profile of the received reflected response signal with a predetermined response signal profile and to control, based on the comparison, the electrical circuitry (7) in order to control an operational state of the vaporizer (5, 6).

2. The aerosol generating system (V) according to claim 1, further comprising:
an authorization trigger module (4b) coupled to the authorization controller (8), the authorization trigger module (4b) being configured to detect a user-initiated activation condition for the aerosol generating system (V) and to activate the authorization controller (8) upon detection of the user-initiated activation condition.

3. The aerosol generating system (V) according to claim 2, wherein the authorization trigger module (4b) includes a mechanical activation element, and the user-initiated activation condition includes an actuation of the mechanical activation element by a user of the aerosol generating system (V).

4. The aerosol generating system (V) according to claim 2, wherein the authorization trigger module (4b) includes an acceleration sensor or a position sensor, the acceleration sensor or position sensor being configured to detect a movement or a position of the aerosol generating system (V), and the user-initiated activation condition includes a detected movement or position of the aerosol generating system (V) corresponding to one of a number of predefined movement profiles or position profiles of the aerosol generating system (V).

5. The aerosol generating system (V) according to claim 2, wherein the authorization trigger module (4b) includes a proximity sensor, the proximity sensor being configured to detect a contact of the aerosol generating system (V) with a body part of a user, and the user-initiated activation condition includes a detected contact of the aerosol generating system (V) with a body part of a user.

6. The aerosol generating system (V) according to one of the claims 1 to 5, further comprising:
an airflow activation sensor (4a) coupled to the authorization controller (8), the airflow activation sensor (4a) being configured to measure an airflow parameter of an airflow (A) streaming through the aerosol generating system (V) and to activate the authorization controller (8) when the measured airflow parameter exceeds a predetermined threshold value.

7. The aerosol generating system (V) according to one of the claims 1 to 6, wherein the signal emitter (9a) comprises an electroacoustic transmitter, an optical emitter or a radar emitter.

8. The aerosol generating system (V) according to claim 7, wherein the signal receiver (9b) comprises an electroacoustic receiver, an optical receiver or a radar receiver.

9. The aerosol generating system (V) according to one of the claims 1 to 8, wherein the signal emitter (9a) and the signal receiver (9b) are arranged within a common transducer module (9).

10. The aerosol generating system (V) according to one of the claims 1 to 9, wherein the authorization controller (8) includes an authorization processor (8a) and a data storage (8b) coupled to the authorization processor (8a), the data storage (8b) being configured to store a plurality of predetermined response signal profiles.

11. The aerosol generating system (V) according to claim 10, wherein the authorization processor (8a) is configured to measure one or more of a frequency of the reflected response signals, a distribution of frequencies of the reflected response signals, and an amplitude of the reflected response signals at a predefined frequency or a predefined set of frequencies.

12. A method (M) for controlling the operation of an aerosol generating system (V), the method (M) comprising:
emitting (M2) an electroacoustic reference signal via an electroacoustic transmitter (9a) of the aerosol generating system (V) into an oral cavity of a user of the aerosol generating system (V);
receiving (M3) reflected response signals of the emitted electroacoustic reference signal via an electroacoustic receiver (9b) of the aerosol generating system (V) from the oral cavity of the user;
comparing (M4) a profile of the received reflected response signals with a predetermined response signal profile stored in the aerosol generating system (V); and
controlling (M5), based on the result of the comparison, an operational state of a vaporizer (5, 6) of the aerosol generating system (V).

13. The method (M) according to claim 12, further comprising:
detecting (M1) a user-initiated activation condition for the aerosol generating system (V) and triggering the emitting (M2) of the electroacoustic reference signal upon detection of the user-initiated activation condition.

14. The method (M) according to claim 13, wherein controlling (M5) the operational state of the vaporizer includes outputting an activation or deactivation signal to the electrical circuitry (7), which signal is maintained until a change in the user-initiated activation condition is detected.

15. Electrical circuitry for an electrically operated aerosol generating system, said electrical circuitry being configured to implement the method of one of the claims 12 to 14.
